# EUROPÄISCHE PATENTANMELDUNG

(11) **EP 0 710 656 A2**
(43) Veröffentlichungstag der Anmeldung: **08.05.1996**
(21) Anmeldenummer: 95113742.1
(22) Anmeldetag: 01.09.1995
(51) Int. Cl.: C07D 251/34, C07C 275/62, C08G 18/38, C09D 175/02, C09J 175/02

(54) **Neue harnstoffgruppenhaltige Polyamine und Verfahren zu ihrer Herstellung**

(30) Priorität: 04.11.1994 DE 4439421
(71) Anmelder: HÜLS AKTIENGESELLSCHAFT, D-45764 Marl (DE)
(72) Erfinder: Gras, Rainer, Dr., D-44879 Bochum (DE); Wolf, Elmar, Dr., D-45661 Recklinghausen (DE)

(57) **Zusammenfassung**

Gegenstand der vorliegenden Erfindung sind niedrigviskose, harnstoffgruppenhaltige (cyclo)-aliphatische Polyamine mit mehr als 2 Aminogruppen sowie Verfahren zu ihrer Herstellung.

## Beschreibung

Gegenstand der vorliegenden Erfindung sind niedrigviskose, harnstoffgruppenhaltige (cyclo)-aliphatische Polyamine mit mehr als 2 Aminogruppen sowie Verfahren zu ihrer Herstellung.

Niedermolekulare, Harnstoffgruppen enthaltende (cyclo)-aliphatische Diamine, erst recht natürlich höhere funktionelle Polyamine, sind bei Raumtemperatur entweder fest oder hochviskos und lösemittelfrei nicht verarbeitbar.

Überraschenderweise konnten niedrigviskose, harnstoffgruppenhaltige (cyclo)-aliphatische Polyamine erhalten werden, indem man (cyclo)-aliphatische Diamine mit Isocyanuratgruppen bzw. Biuretgruppen enthaltenden Polyisocyanaten umsetzte.

Gegenstand der vorliegenden Erfindung sind somit niedrigviskose, harnstoffgruppenhaltige (cyclo)-aliphatische Polyamine mit mehr als zwei Aminogruppen, erhältlich durch Umsetzung von Diaminen der Formel I
I. R¹ ― NH ― A ― NH ― R²,
   wobei A einen (cyclo)-aliphatischer Kohlenwasserstoffrest mit 2 - 16 C-Atomen, der gegebenfalls alkylsubstituiert ist, darstellt, R¹ und R² gleich H oder einen Rest der Formel 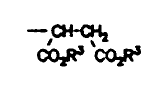 und R³ einen (cyclo)-aliphatischen Kohlenwasserstoffrest mit 1 - 12 C-Atomen, der gegebenfalls verzweigt ist, bedeuten,
   mit Isocyanurat- und/oder Biuretgruppen enthaltenden Polyisocyanaten der Formel II
II.
   a) OCN-(B)-[X]ₙ-NCO
      und
   b) OCN-(B)-NCO,
   wobei B
   -(CH₂)₆-; 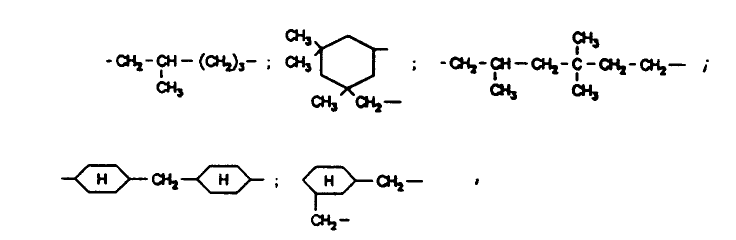 [X]ₙ 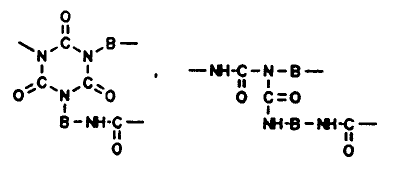 und n gleich 1 - 4
   bedeuten, das Gewichtsverhältnis a zu b gleich (100 - 20) zu (0 - 80) beträgt und pro NCO-Gruppe 1 bis 5 mol der Diamine I eingesetzt werden.

Weiterhin Gegenstand der Erfindung sind niedrigviskose, harnstoffgruppenhaltige (cyclo)-aliphatische Polyamine mit mehr als zwei Aminogruppen, erhältlich durch Umsetzung von Diaminen der Formel I
I. NH₂ ― A ― NH₂,
   wobei A einen (cyclo)-aliphatischer Kohlenwasserstoffrest mit 2 - 16 C-Atomen, der gegebenfalls alkylsubstituiert ist, darstellt,
   mit Isocyanurat- und/oder Biuretgruppen enthaltenden Polyisocyanaten der Formel II
II.
   a) OCN-(B)-[X]ₙ-NCO
      und
   b) OCN-(B)-NCO,
   wobei B
   -(CH₂)₆-; 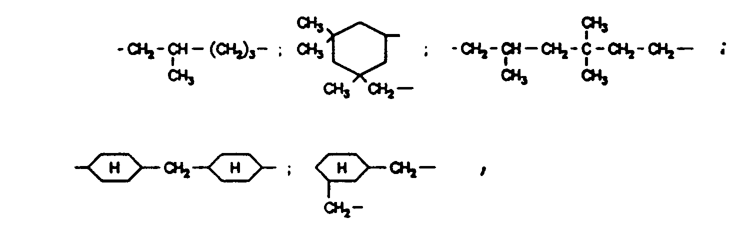 [X]ₙ 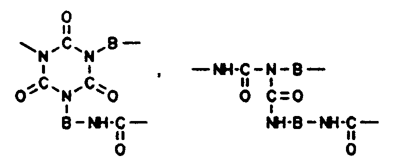 und n gleich 1 - 4
   bedeuten, das Gewichtsverhältnis a zu b gleich (100 - 20) zu (0 - 80) beträgt und pro NCO-Gruppe 1 bis 5 mol der Diamine I eingesetzt werden,
III. sowie anschließende Umsetzung mit einem Mol Malein- oder Fumarsäureester pro NH₂-Äquivalent.

Ebenfalls Gegenstand der Erfindung sind niedrigviskose, harnstoffhaltige (cyclo)-aliphatische Polyamine, wobei diese inerte Verbindungen wie z. B. Weichmacher in Mengen von 5 bis 100 Gewichtsteile, bezogen auf die Komponente I, enthalten.

Die erfindungsgemäßen niedrigviskosen, harnstoffgruppenhaltigen (cyclo)-aliphatischen Polyamine können nach verschiedenen Verfahren, gemäß den Ansprüchen 5 und 6, erhalten werden.

Im Falle für R¹ und R² gleich Wasserstoff oder ein Rest der oben angegebenen Formel - erste Variante - erfolgt die Herstellung der erfindungsgemäßen Verbindungen dermaßen, daß die Komponenten I und II im Molverhältnis von 1 bis 5 : 1 umgesetzt werden, wobei zu dem auf 100 - 190°C erhitzten Diamin I das Polyisocyanat II unter intensiver Rührung und Stickstoffabdeckung portionsweise zugegeben wird. Nach beendeter Polyisocyanatzugabe wird das Reaktionsgemisch noch ca. 10 min. weiter erhitzt. Danach wird auf Raumtemperatur abgekühlt.

Eine zweite Variante zur Herstellung der erfindungsgemäßen Polyamine besteht darin, daß man die Polyamine der ersten Variante mit R¹ und R² gleich Wasserstoff wie folgt umsetzt.

Die auf Raumtemperatur abgekühlte Reaktionsmischung wird mit Malein- bzw. Fumarsäureester so umgesetzt, daß auf 1 NH₂-Äquivalent 1 mol Malein- bzw. Fumarsäureester bei 50 bis 70 °C zur Reaktion kommen.

Die erfindungsgemäßen Verbindungen - die Reaktionsprodukte aus I und II - sind gekennzeichnet durch einen Amin-Gehalt von 1 - 4 mmol/g, durch einen Isocyanuratgruppen-Gehalt von 1 - 6 % (bezogen auf 126 g/mol), bzw. durch einen Biuretgruppen-Gehalt von 1 - 5 % (bezogen auf 98 g/mol). Ihre Viskosität bei 25 °C ist in einem weiten Bereich variierbar. Je nach NH : : NCO-Äquivalenzverhältnis beträgt sie 100 - 10⁵ mPa.s.

Die zur Herstellung der erfindungsgemäßen Polyamine eingesetzten Diamine I sind seit langem bekannt und nicht Gegenstand der vorliegenden Erfindung. Sie werden durch Umsetzung der Diamine H₂N-A-NH₂ und Maleinsäure- bzw. Fumarsäureester erhalten, wobei pro Mol Malein- bzw. Fumarsäureester 0,5 Mol Diamin in bekannter Weise zur Reaktion kommen. Bei den Diaminen H₂N-A-NH₂ handelt es sich um aliphatische bzw. cycloaliphatische Diamine, wie z. B. 1.6-Diaminohexan, 2-Methyl-1.5-diaminopentan, 2.2.4 (2.4.4)-Trimethyl-1.6-diaminohexan (TMD), 2.4'- und/oder 4.4'-Diamino-dicyclohexylmethan, 1.11-Diaminoundecan, 1.12-Diaminododecan, 1.4- bzw. 1.2-Diaminocyclohexan, m-Hexahydroxylylendiamin, 1-Amino-3.5.5-trimethyl-5-aminomethyl-cyclohexan (Isophorondiamin, abgekürzt: IPD).

Die zur Herstellung der erfindungsgemäßen Polyamine geeigneten Polyisocyanate sind Isocyanurat- oder Biuretgruppen enthaltende Polyisocyanate. Die Herstellung dieser Isocyanuratoisocyanate wird in den DE-OSS 23 25 826, 26 44 684, 28 21 109, 29 16 301 beschrieben. Ihre Herstellung erfolgt derart, daß das Diisocyanat mit Hilfe eines Katalysators - am besten haben sich die in der DE-OS 29 16 201 beschriebenen quaternären Ammoniumsalze erwiesen - partiell trimerisiert wird, und anschließend das nicht umgesetzte Diisocyanat durch eine Dünnschichtdestillation vom Reaktionsprodukt, dem Isocyanuratgruppen enthaltenden Polyisocyanat, abgetrennt wird. In der Regel werden für die Umsetzung der Diamine mit den Isocyanuratoisocyanaten nach dem erfindungsgemäßen Verfahren die monomerbefreiten Isocyanuratoisocyanate eingesetzt. In machen Fällen hat es sich als zweckmäßig erwiesen, das partiell trimerisierte Diisocyanatgemisch ohne vorherige Abtrennung des nicht umgesetzten Diisocyanats einzusetzen. Die erfindungsgemäßen geeigneten Biuretgruppen enthaltenden Polyisocyanate werden in der DE-OS 23 08 015 beschrieben. Ihre Herstellung erfolgt durch Umsetzung von Wasser mit überschüssigem Diisocyanat, wobei nach erfolgter Biuretbildung das nicht umgesetzte Diisocyanat durch Dünnschichtdestillation abgetrennt. Hier gilt das gleiche wie bei den Isocyanuratgruppen enthaltenden Polyisocyanaten. In der Regel wird das monomerbefreite Biuretgruppen enthaltende Polyisocyanat eingesetzt; in manchen Fällen kann aber auf eine Diisocyanatabtrennung verzichtet werden.

Die erfindungsgemäßen Verbindungen eignen sich in hervorragender Weise zur Herstellung von PUR-Reaktionslacken, Beschichtungen und Klebstoffen, deren Aushärtung bei Raumtemperatur oder etwas erhöhter Temperatur, in der Regel 80 °C, erfolgt.

Die folgenden Beispiele sollen die Erfindung näher erläutern.

### A. 1. Nichterfindungsgemäße Verbindungen (Reaktionsprodukte aus 1 mol Diamin und 2 mol Maleinsäureester)

### Allgemeine Herstellungsvorschrift

Zu 2 mol Malein- bzw. Fumarsäureester wird bei 50 - 60 °C 1 mol Diamin so zugetropft, daß die Temperatur des Reaktionsgemisches nicht über 70 °C steigt. Nach Beendigung der Diaminzugabe wird zur Vervollständigung der Reaktion noch ca. 2 h bei 60 °C weiter erhitzt. Für die weitere Umsetzung mit dem Polyisocyanat ist eine weitere Behandlung (Destillation) des Reaktionsgemisches nicht erforderlich.

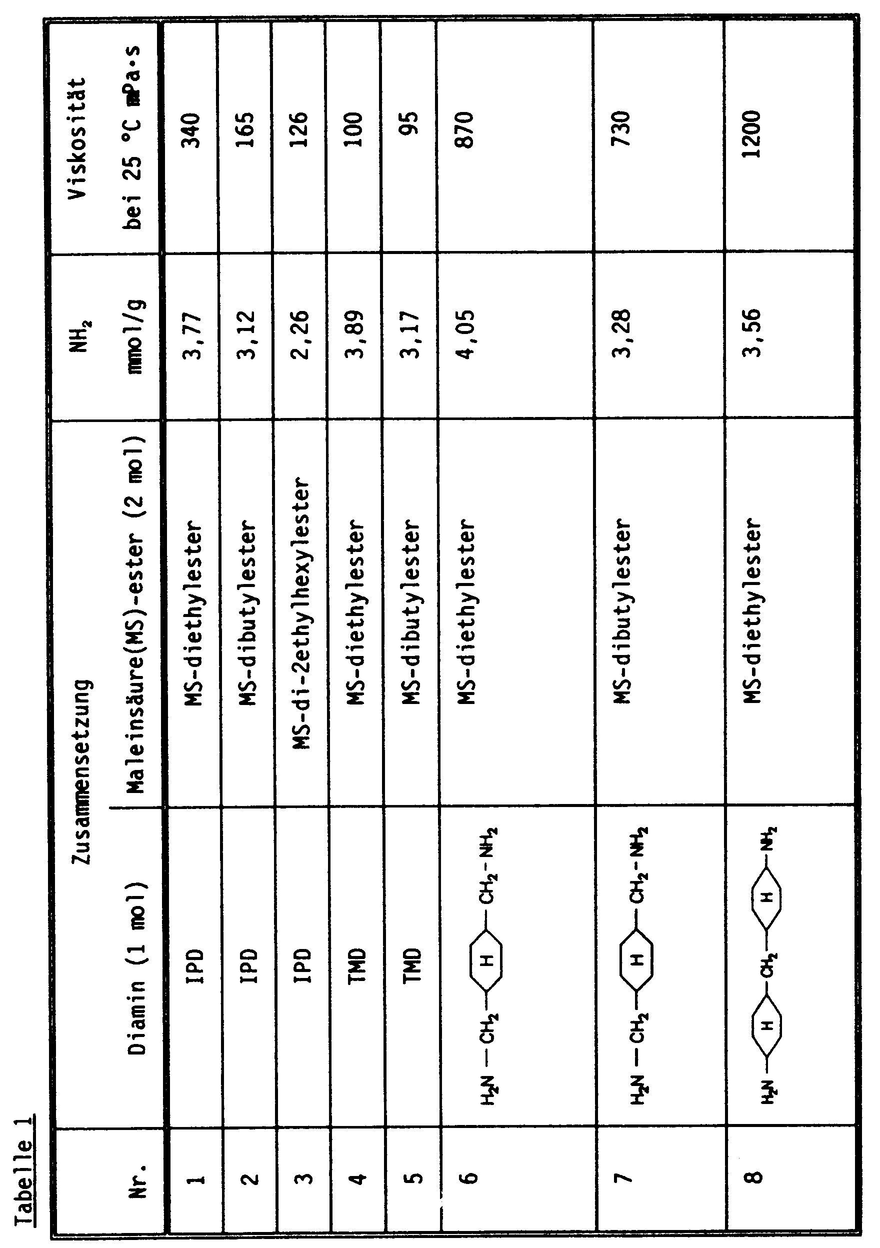

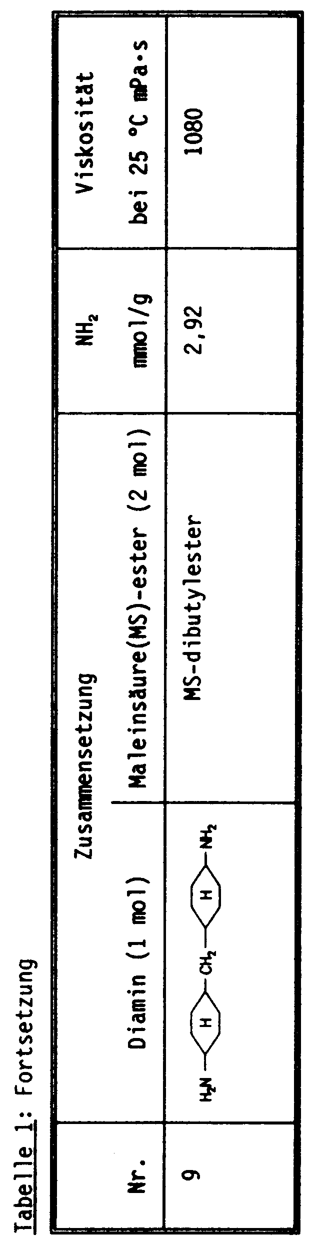

### A. 2. Polyisocyanate

### Polyisocyanat 1:

Handelsübliches Isocyanuratoisocyanat auf Basis von Isophorondiisocyanat mit einem NCO-Gehalt von 17,2 %.

### Polyisocyanat 2:

Handelsübliches Isocyanuratoisocyanat auf Basis von Hexamethylendiisocyanat mit einem NCO-Gehalt von 21 %.

### Polyisocyanat 3:

Handelsübliches Biuret auf Basis von Hexamethylendiisocyanat mit einem NCO-Gehalt von 22,9 %.

### B. 1. Beispiele 1 bis 11

### Erfindungsgemäße Verbindungen (Umsetzung: A. 1. + A.2)

### Allgemeine Herstellungsvorschrift

Zu dem auf 100 - 150 °C erhitzten disekundären Diamin wird das Polyisocyanat unter intensiver Rührung und Stickstoffabdeckung portionsweise innerhalb von ca. 0,5 h zugegeben. Nach Beendigung der Polyisocyanatzugabe wird sofort auf Raumtemperatur abgekühlt.

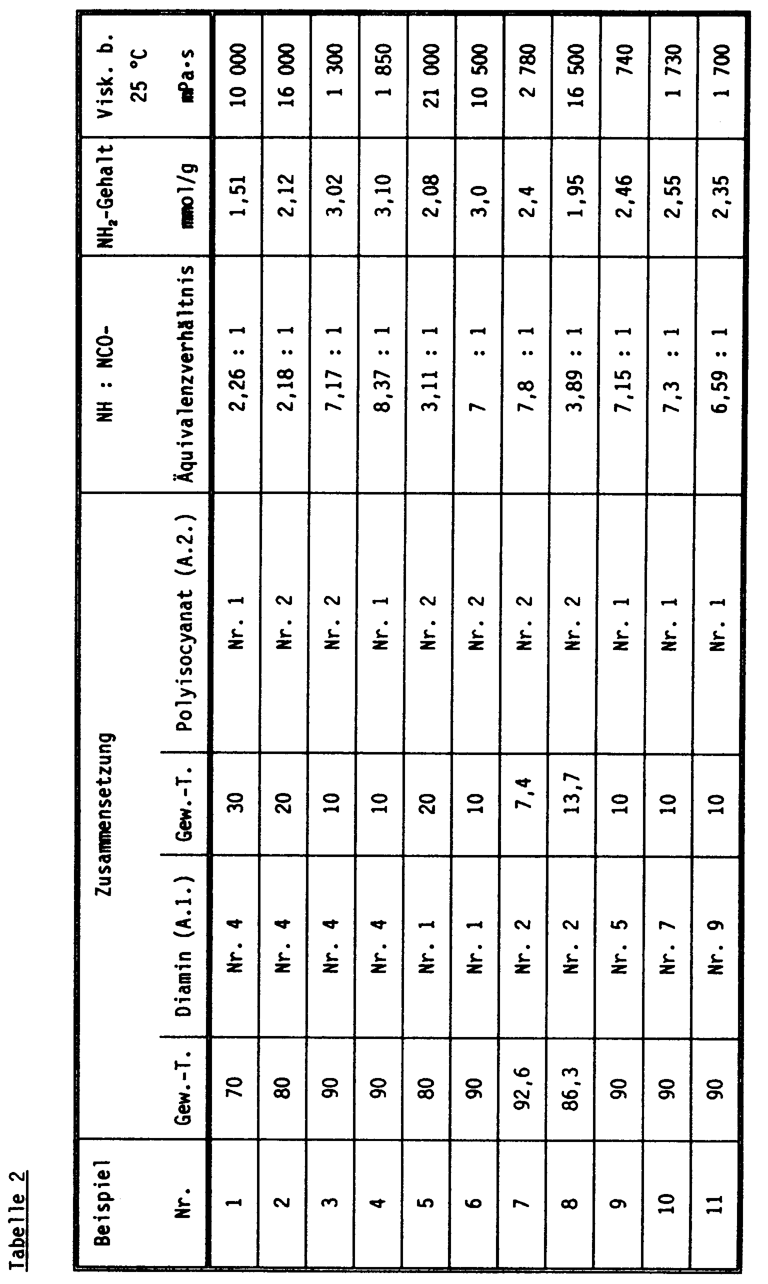

### B. 2. Beispiele 12 bis 25

### Erfindungsgemäße Verbindungen (Reaktionsprodukte aus Diaminen und Polyisocyanaten (A.2.))

### Allgemeine Herstellungsvorschrift

Zu dem auf 150 - 180 °C erhitzten Diamin wird das Polyisocyanat unter intensiver Rührung und Stickstoffabdeckung portionsweise so zugegeben, daß die Temperatur des Reaktionsgemisches nicht über 190 °C steigt. Nach Beendigung der Polyisocyanatzugabe wird noch ca. 10 min. weiter erhitzt. Danach ist die Reaktion beendet.

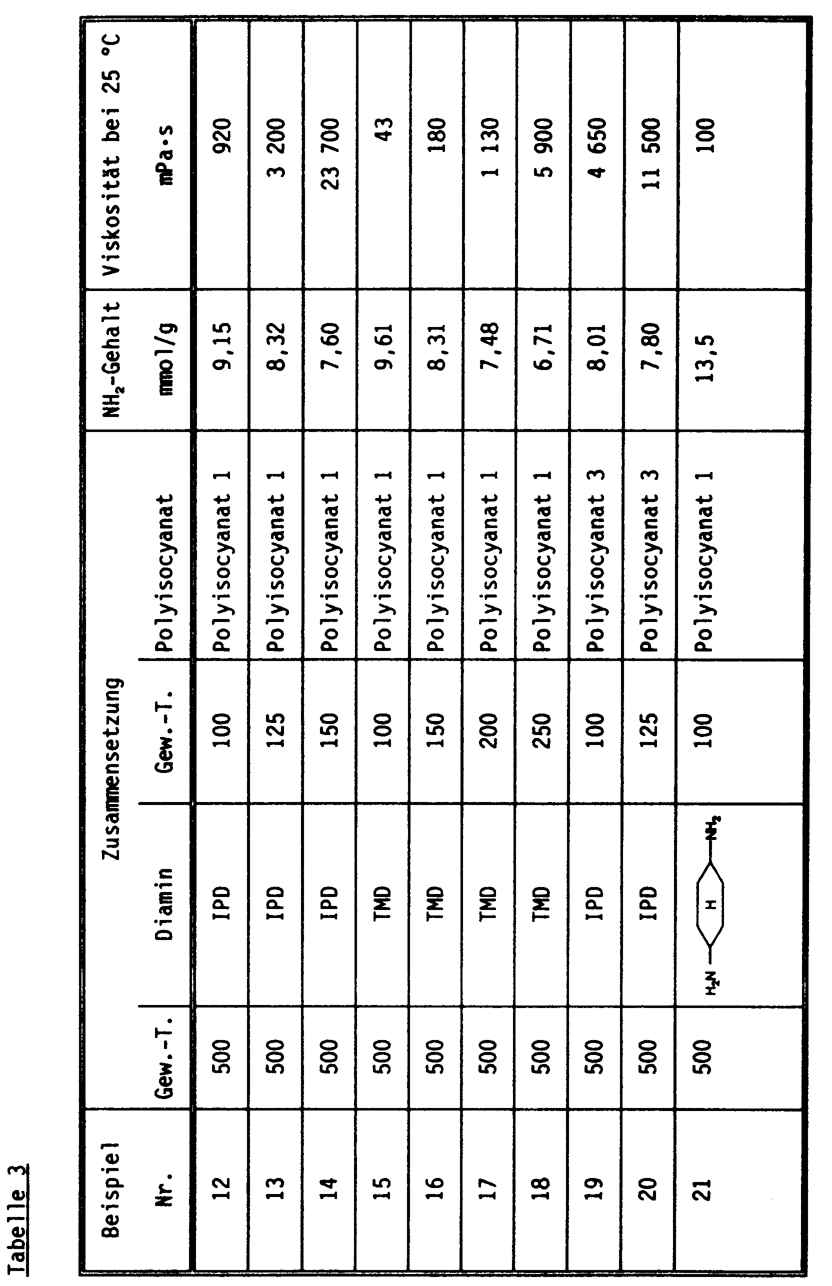

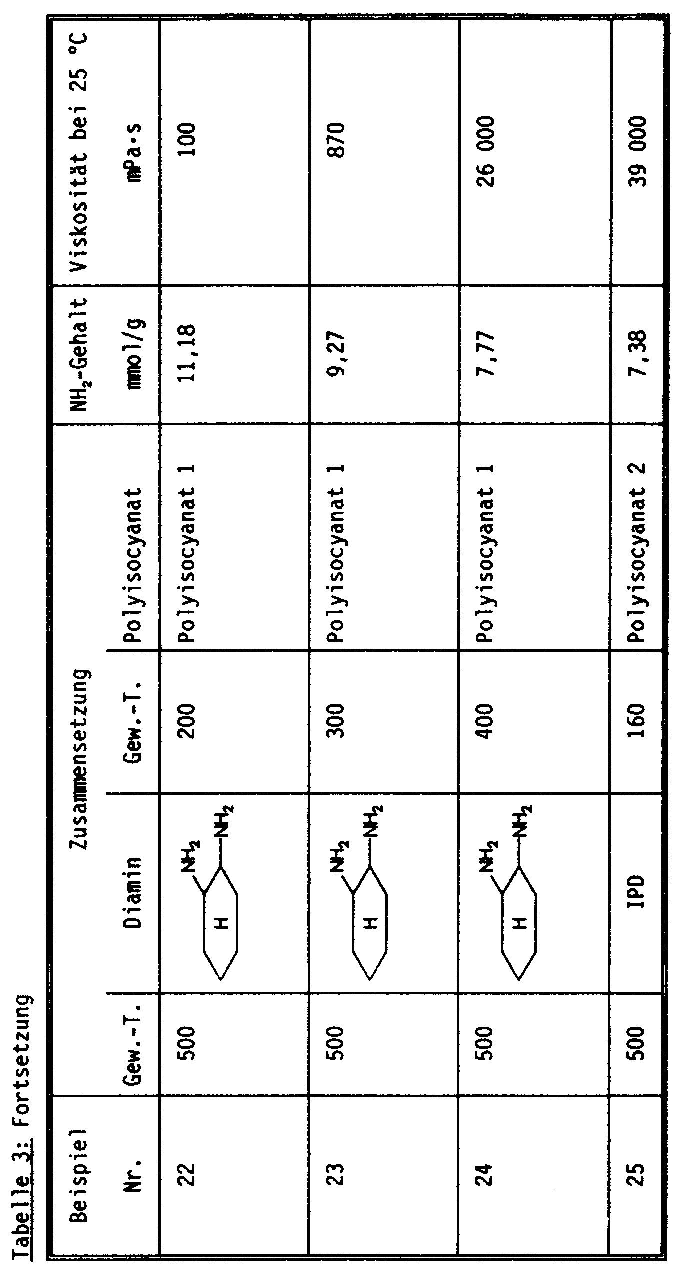

### B.3. Beispiele 25 bis 30

### Erfindungsgemäße Verbindungen (Reaktionsprodukte aus B.2. + Maleinsäureester)

### Allgemeine Herstellungsvorschrift

Die Herstellung der erfindungsgemäßen Verbindungen erfolgt in Analogie zu der Herstellung der in A.1. beschriebenen Verbindungen, wobei auf 1 Mol Malein- bzw. Fumarsäureester 1 Amin-Äquivalent der Amine B.2. zur Reaktion kommt.

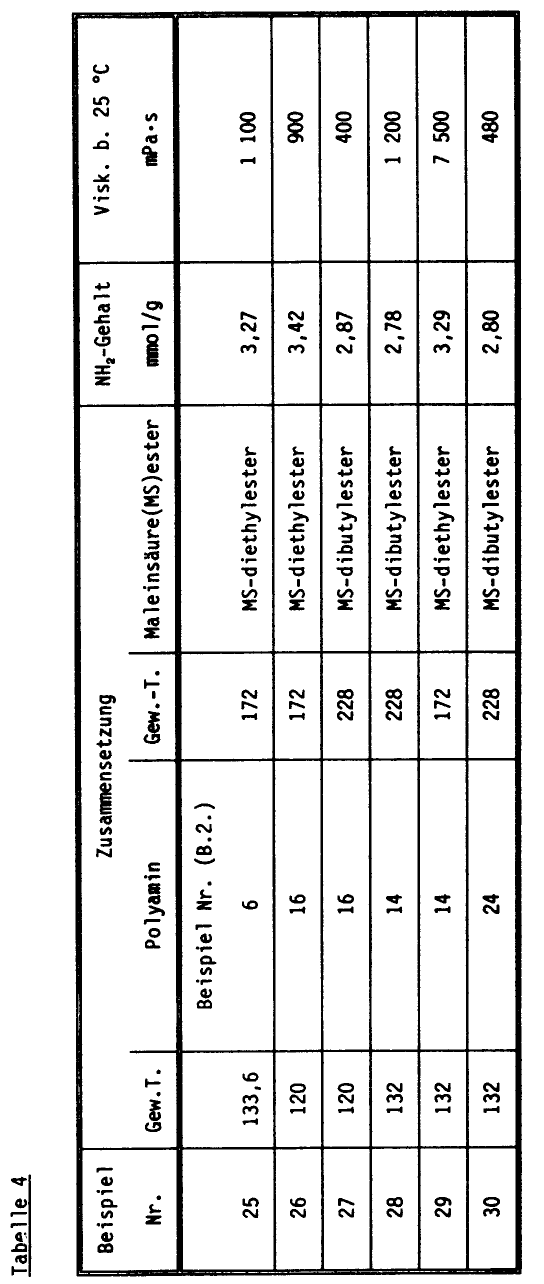

## Patentansprüche

1. Niedrigviskose, harnstoffgruppenhaltige (cyclo)-aliphatische Polyamine mit mehr als zwei Aminogruppen,
erhältlich durch Umsetzung von Diaminen der Formel I
I. R¹ ― NH ― A ― NH ― R²,
wobei A einen (cyclo)-aliphatischer Kohlenwasserstoffrest mit 2 - 16 C-Atomen, der gegebenfalls alkylsubstituiert ist, darstellt, R¹ und R² gleich H oder einen Rest der Formel 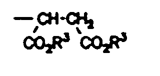 und R³ einen (cyclo)-aliphatischen Kohlenwasserstoffrest mit 1 - 12 C-Atomen, der gegebenfalls verzweigt ist, bedeuten,
mit Isocyanurat- und/oder Biuretgruppen enthaltenden Polyisocyanaten der Formel II
II.
a) OCN-(B)-[X]ₙ-NCO
und
b) OCN-(B)-NCO,
wobei B
-(CH₂)₆-; 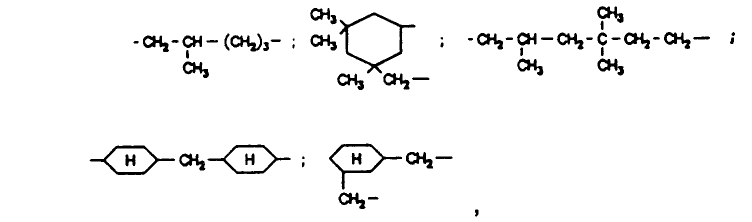 [X]ₙ 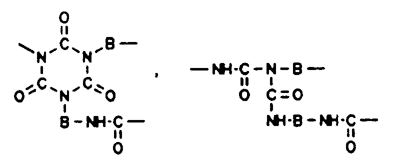 und n gleich 1 - 4
bedeuten, das Gewichtsverhältnis a zu b gleich (100 - 20) zu (0 - 80) beträgt und pro NCO-Gruppe 1 bis 5 mol der Diamine I eingesetzt werden.

2. Niedrigviskose, harnstoffgruppenhaltige (cyclo)-aliphatische Polyamine mit mehr als zwei Aminogruppen,
erhältlich durch Umsetzung von Diaminen der Formel I
I. NH₂ ― A ― NH₂,
wobei A einen (cyclo)-aliphatischer Kohlenwasserstoffrest mit 2 - 16 C-Atomen, der gegebenfalls alkylsubstituiert ist, darstellt,
mit Isocyanurat- und/oder Biuretgruppen enthaltenden Polyisocyanaten der Formel II
II.
a) OCN-(B)-[X]ₙ-NCO
und
b) OCN-(B)-NCO,
wobei B
-(CH₂)₆-; 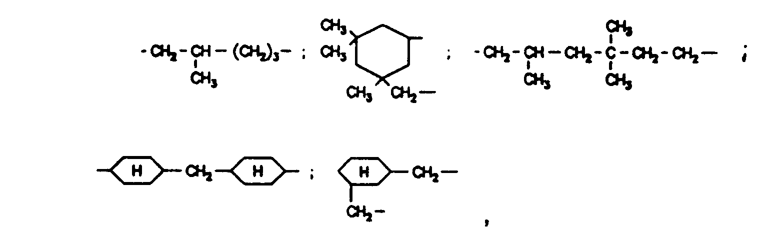 [X]ₙ 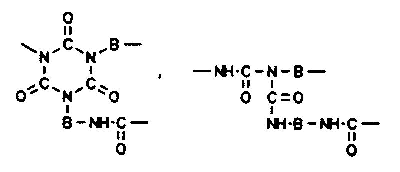 und n gleich 1 - 4
bedeuten, das Gewichtsverhältnis a zu b gleich (100 - 20) zu (0 - 80) beträgt und pro NCO-Gruppe 1 bis 5 mol der Diamine I eingesetzt werden,
III. sowie anschließende Umsetzung mit einem Mol Malein- oder Fumarsäureester pro NH₂-Äquivalent.

3. Polyamine nach Anspruch 1 oder 2,
dadurch gekennzeichnet,
daß sie 0,01 bis 5 Gew.-Teile inerte Verbindungen enthalten.

4. Polyamine nach Anspruch 3,
dadurch gekennzeichnet,
daß sie Weichmacher enthalten.

5. Verfahren zur Herstellung von niedrigviskosen, harnstoffgruppenhaltigen (cyclo)-aliphatischen Polyaminen,
dadurch gekennzeichnet,
daß sie erhalten werden durch Umsetzung von Diaminen
I. R¹ - NH - A - NH - R²,
wobei A einen (cyclo)-aliphatischer Kohlenwasserstoffrest mit 2 - 16 C-Atomen, der gegebenfalls alkylsubstituiert ist, darstellt, und R¹ und R² gleich H oder einen Rest der Formel 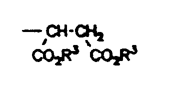 und R³ einen (cyclo)-aliphatischen Kohlenwasserstoffrest mit 1 - 12 C-Atomen, der gegebenfalls verzweigt ist, bedeuten,
mit Isocyanurat- und/oder Biuretgruppen enthaltenden Polyisocyanaten
II.
a) OCN-(B)-[X]ₙ-NCO
und
b) OCN-(B)-NCO,
wobei B
-(CH₂)₆-; 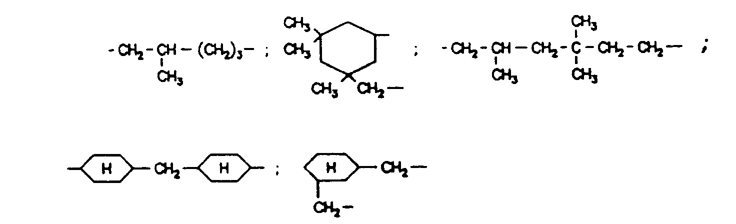 und [X]ₙ 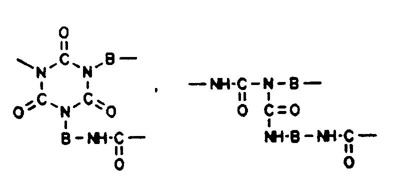 n gleich 1 - 4
bedeuten, das Gewichtsverhältnis a zu b gleich (100 - 20) zu (0 - 80) beträgt und pro NCO-Gruppe 1 bis 5 mol der Diamine I eingesetzt werden, bei Temperaturen von 100 bis 190 °C.

6. Verfahren zur Herstellung von harnstoffhaltigen (cyclo)-aliphatischen Polyaminen,
dadurch gekennzeichnet,
daß sie erhalten werden durch Umsetzung von Diaminen
I. H₂N - A - NH₂,
wobei A einen (cyclo)-aliphatischer Kohlenwasserstoffrest mit 2 - 16 C-Atomen, der gegebenfalls alkylsubstituiert ist, darstellt,
mit Isocyanurat- und/oder Biuretgruppen enthaltenden Polyisocyanaten
II.
a) OCN-(B)-[X]ₙ-NCO
und
b) OCN-(B)-NCO,
wobei B
-(CH₂)₆-; 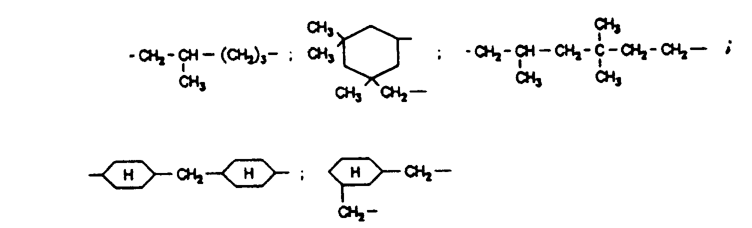 und [X]ₙ 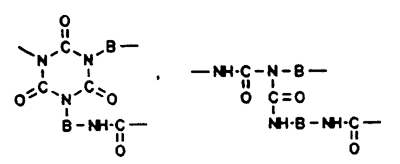 n gleich 1 - 4
bedeuten, das Gewichtsverhältnis a zu b gleich (100 - 20) zu (0 - 80) beträgt und pro NCO-Gruppe 1 bis 5 mol der Diamine I eingesetzt werden, bei Temperaturen von 150 - 190 °C,
III. und anschließende Umsetzung mit einem Mol Malein- oder Fumarsäureester pro NH₂-Äquivalent bei 50 bis 70 °C.

7. Verwendung der harnstoffhaltigen (cyclo)-aliphatischen Polyamine in PUR-Reaktionslacken, sowie als Komponenten für Beschichtungs- und Klebstoffzusammensetzungen.
